# EUROPÄISCHE PATENTANMELDUNG

(11) **EP 4 115 872 A1**
(43) Veröffentlichungstag der Anmeldung: **11.01.2023**
(21) Anmeldenummer: 22177162.9
(22) Anmeldetag: 03.06.2022
(51) Int. Cl.: A61K 8/03, A61K 8/34, A61K 8/37, A61K 8/41, A61Q 5/00, A61Q 5/12

(54) **TRANSFORMIERENDES 2-PHASEN-HAARPFLEGEMITTEL**

(30) Priorität: 06.07.2021 DE 102021207052
(71) Anmelder: Henkel AG & Co. KGaA, 40589 Düsseldorf (DE)
(72) Erfinder: Ingwersen, Jasmin, 22547 Hamburg (DE); Ahnfeldt, Tina, 23815 Strukdorf (DE); Westphal, Petra, 21629 Neu Wulmstorf (DE); Kerl, Sylvia, 25474 Bönningstedt (DE)

(57) **Zusammenfassung**

Die vorliegende Erfindung betrifft Haarbehandlungsmittel für die Pflege von Haaren in Form eines Zweiphasensystems mit zwei separaten Schichten, enthaltend C2-C6-Monohydroxyalkohol(e), C2-C12-Polyol(e), C8-C24-Alkohol(e), C6-C24-Fettsäureester und (ein) kationische(s) Tensid(e), die bei Wasserkontakt eine Creme, eine Lotion oder ein Gel transformieren.

## Beschreibung

Die vorliegende Erfindung betrifft Haarbehandlungsmittel für die Pflege von Haaren in Form eines Zweiphasensystems mit zwei separaten Schichten, umfassend eine Wirkstoffkombination aus spezifischen Alkoholen und Polyolen, Fettsäureestern und kationischen Tensiden. Die Erfindung betrifft weiterhin ein Verfahren zur Pflege von Haaren unter Verwendung der Haarbehandlungsmittel.

Mit der Zeit und insbesondere bei längeren Haaren treten Schädigungen der Haarfasern auf. Unter Schädigungen werden sowohl durch Reibungsschäden wie Kämmen, Bürsten, Toupieren verursachte Schädigungen als auch strukturelle oder chemische Veränderungen der Haarfasern verstanden, welche durch Prozesse wie Färben, Bleichen oder permanente Verformung verursacht werden können. Des Weiteren können auch Sonneneinwirkung, Umwelteinflüsse oder (Hitze)-Styling die Haarfasern schädigen.

Trockene oder geschädigte Haare können mit üblichen Leave-on- oder Rinse-off-Pflegeprodukten behandelt werden, was zu einer signifikanten Verbesserung der inneren und äußeren Haarstruktur beiträgt.

Die Bedürfnisse der Verbraucher und auch das Know-How der Kosmetikhersteller verändern bzw. verbessern sich jedoch und erfordern oder ermöglichen eine stetige Verbesserung und Anpassung existierender Haarpflegeprodukte.

Im Markt erhältliche Haarpflegeprodukte umfassen meist eine Kombination verschiedener Pflegewirkstoffe wie beispielsweise kationische Tenside und Polymere, Öle unterschiedlicher Polarität, Silikone etc., die in einer gewöhnlich wässrigen Basis aufwändig stabilisiert werden müssen. Dafür ist einerseits ein höherer Energieaufwand unumgänglich und andererseits erfordert die Stabilisierung insbesondere wasserunlöslicher Pflegewirkstoffe den zusätzlichen Einsatz synthetischer Hilfsstoffe, welche zunehmend unerwünscht sind.

Es besteht das Bedürfnis nach wenig komplexen, innovativen, schnell wirksamen, einfach sowie vielseitig anwendbaren Applikationsformen für die Haarpflege mit hohem Haarpflegepotential. Ziel der vorliegenden Erfindung war die Bereitstellung solcher Haarpflegeformulierungen.

Das Ziel wird erfindungsgemäß durch ein Haarbehandlungsmittel für die Pflege von Haaren in Form eines Zweiphasensystems mit zwei separaten Schichten gelöst, welches - bezogen auf sein Gesamtgewicht -
a) 10-40 Gew.-% mindestens eines C2-C6-Monohydroxyalkohols,
b) 10 - 60 Gew.-% mindestens eines C2-C12-Polyols,
c) 5 - 35 Gew.-% mindestens eines C8-C24-Alkohols,
d) 5 - 35 Gew.-% mindestens eines C6-C24-Fettsäureesters und
e) 0,1 - 7 Gew.-% mindestens eines kationischen Tensids enthält.

Die erfindungsgemäßen Haarbehandlungsmittel weisen den Vorteil auf, dass sie in Form lagerstabiler Zweiphasensysteme vorliegen, die sich ressourcenschonend herstellen, verpacken und transportieren lassen, denn sie enthalten noch keinen bzw. nur einen geringen Gehalt an Wasser. Die beiden Schichten der erfindungsgemäßen Haarbehandlungsmittel lassen sich durch Schütteln schnell und gründlich durchmischen und trennen sich im Ruhezustand wieder in zwei separate Schichten auf.

Die erfindungsgemäßen Haarbehandlungsmittel weisen sowohl im Ruhezustand als auch nach der Homogenisierung durch Schütteln eine dünnflüssige Konsistenz auf, wodurch sie sich einfach und rückstandsarm aus einer Verpackung entnehmen sowie schnell und sehr gut bis in die Haarspitzen verteilen lassen. In Kombination mit Wasser transformieren sie in eine Creme, Lotion oder ein Gel, welche(s) selbst bei einer kurzen Einwirkungszeit auf den Haaren haftet und eine gute Abscheidung der Pflegewirkstoffe begünstigt.

Die erfindungsgemäßen Haarbehandlungsmittel sind insbesondere geeignet für die Verwendung als Rinse-off-Zusammensetzung.

Mit erfindungsgemäßen Zusammensetzungen behandelte Haare weisen ein gesundes, gepflegtes Erscheinungsbild und eine angenehme Haptik auf. Insbesondere lassen sie sich gut entwirren und weisen eine sehr gute Kämmbarkeit im nassen und trockenen Haar, ein gepflegtes und geschmeidiges Haargefühl bis in die Haarspitzen, strahlenden Glanz und Anti-Frizz auf. Eine Beschwerung der Haare konnte auch bei regelmäßiger Anwendung der erfindungsgemäßen Zusammensetzungen nicht beobachtet werden.

Die erfindungsgemäßen Haarbehandlungsmittel können nach erfolgter Homogenisierung unmittelbar nach der Haarreinigung oder nach der Reinigung und Pflege mit einem üblichen Konditioniermittel auf die Haare aufgetragen werden. Alternativ können die erfindungsgemäßen Haarbehandlungsmittel auch vor der Haarreinigung als Vorbehandlungsmittel verwendet werden. Die erfindungsgemäßen Haarbehandlungsmittel können auf die handtuchtrockenen und/oder feuchten Keratinfasern aufgetragen werden.

Die Erfindung betrifft ferner ein Haarbehandlungsmittel wie zuvor beschrieben, umfassend (bezogen auf das Gesamtgewicht des Haarbehandlungsmittels):
a) 10 bis 40 Gew.-% mindestens eines C2-C6-Monohydroxyalkohols.

C2-C6-Monohydroxyalkohole a) unterstützen die Auflösung oder Solubilisierung wasserunlöslicher Komponenten in den erfindungsgemäßen Haarbehandlungsmitteln, so dass vorzugsweise Zweiphasenzusammensetzungen bereitgestellt werden können, in denen mindestens eine der beiden Schichten transparent ist. Eine zusätzliche Stabilisierung - wie bei herkömmlichen, emulsionsbasierten Zusammensetzungen üblich - ist nicht erforderlich.

Geeignete C2-C6-Monohydroxyalkohole a) können ausgewählt sein aus Ethanol, n-Propanol, Isopropanol, n-Butanol, Isobutanol, 2-Methyl-2-butanol, Pentanol, Hexanol oder Mischungen dieser Alkohole. Bevorzugt sind Ethanol, n-Propanol, Isopropanol und Mischungen dieser Alkohole. Insbesondere bevorzugt ist Ethanol.

Der mindestens eine C2-C6-Monohydroxyalkohol a) kann in den erfindungsgemäßen Haarbehandlungsmitteln vorzugsweise in Mengen von 11 bis 38 Gew.-%, mehr bevorzugt 12 bis 37 Gew.-%, besonders bevorzugt von 13 bis 36 Gew.-% und insbesondere von 15 bis 35 Gew.-% eingesetzt werden (bezogen auf das Gesamtgewicht der Haarbehandlungsmittel). Vorzugsweise enthalten erfindungsgemäße Haarbehandlungsmittel Ethanol in den zuvor genannten Mengen. Für einige Anwendungsformen kann es von Vorteil sein, Mischungen von C2-C6-Monohydroxyalkoholen a) in den zuvor genannten Mengen einzusetzen.

Die Erfindung betrifft ferner ein Haarbehandlungsmittel wie zuvor beschrieben, umfassend (bezogen auf das Gesamtgewicht des Haarbehandlungsmittels):
b) 10 bis 60 Gew.-% mindestens eines C2-C12-Polyols.

Diese Polyole dienen unter anderem als Feuchthaltemittel. Ihr Ziel ist es, (Kopf)haut und Haare optimal mit Feuchtigkeit zu versorgen.

Für eine erfolgreiche Transformierung der erfindungsgemäßen Mittel in eine Creme, Lotion oder in ein Gel hat es sich als vorteilhaft erwiesen, wenn als b) eine Mischung bestimmter Diole und Polyole (umfassend mindestens drei Hydroxylgruppen) eingesetzt wird. Zudem kann durch die Steuerung des Polyolgehalts in einer solchen Mischung das Erscheinungsbild der erfindungsgemäßen Zusammensetzungen (d.h. das Vorliegen zweier optisch erkennbarer Schichten) kontrolliert werden.

Eine bevorzugte Ausführungsform der erfindungsgemäßen Haarbehandlungsmittel ist demnach dadurch gekennzeichnet, dass sie als C2-C12-Polyol b) eine Mischung aus
bi) mindestens einem C2-C6-Diol und
bii) mindestens einem C2-C12-Polyol, welches mindestens drei Hydroxylgruppen aufweist, umfassen, wobei das Gewichtsverhältnis i) : ii) von 1 : 10 bis 1 : 3 beträgt.

Eine weitere bevorzugte Ausführungsform der erfindungsgemäßen Haarbehandlungsmittel ist dadurch gekennzeichnet, dass sie - bezogen auf ihr Gesamtgewicht -
bi) 1-15 Gew.-% mindestens eines C2-C6-Diols und
bii) 10-35 Gew.-% mindestens eines C2-C12-Polyols, welches mindestens drei Hydroxylgruppen aufweist, enthalten.

Bevorzugte Haarbehandlungsmittel im Sinne der vorliegenden Erfindung sind außerdem dadurch gekennzeichnet, dass das mindestens eine C2-C6-Diol bi) ausgewählt wird aus mindestens einem Diol der Gruppe, die gebildet wird aus 1,2-Propylenglykol, Butan-1,3-diol, Butan-2,3-diol, 1-Methyl-2,4-butandiol, 2-Methyl-2,4-pentandiol, 1,2-Pentandiol, 1,5-Pentandiol, 2-Ethyl-1,3-hexandiol. Dabei ist 1,2-Propylenglykol besonders bevorzugt.

Besonders bevorzugte Haarbehandlungsmittel enthalten das mindestens eine C2-C6-Diol bi) in einem Gewichtsanteil von 1 - 15 Gew.-%, mehr bevorzugt 1,5 - 14 Gew.-%, besonders bevorzugt 2-13 Gew.-%, ganz besonders bevorzugt 2,5 - 12 Gew.-% und insbesondere 3 bis 11 Gew.-% am Gesamtgewicht des Haarbehandlungsmittels.

Ganz besonders bevorzugte Haarbehandlungsmittel enthalten 1,2-Propylenglykol in den zuvor genannten Mengen.

Weiterhin besonders bevorzugte Haarbehandlungsmittel im Sinne der vorliegenden Erfindung sind außerdem dadurch gekennzeichnet, dass das mindestens eine C2-C12-Polyol bii) ausgewählt wird aus mindestens einem Polyol der Gruppe, die gebildet wird aus Glycerin, 1,2,4-Pentantriol, 1,3,4-Pentantriol, 1,3,5-Pentantriol, Sorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Diglycerin, Polyglycerin-3, Glucose, Lactose, Maltose, Polyglycerin-4, Dipropylenglykol, Tripropylenglykol. Dabei ist Glycerin besonders bevorzugt.

Besonders bevorzugte Haarbehandlungsmittel enthalten das mindestens eine C2-C12-Polyol bii) in einem Gewichtsanteil von 10-35 Gew.-%, mehr bevorzugt 12-34 Gew.-%, besonders bevorzugt 13-33 Gew.-%, ganz besonders bevorzugt 14-33 Gew.-% und insbesondere 15 bis 31 Gew.-% am Gesamtgewicht des Haarbehandlungsmittels.

Ganz besonders bevorzugte Haarbehandlungsmittel enthalten Glycerin in den zuvor genannten Mengen.

Die Erfindung betrifft ferner ein Haarbehandlungsmittel wie zuvor beschrieben, umfassend (bezogen auf das Gesamtgewicht des Haarbehandlungsmittels):
c) 5 bis 35 Gew.-% mindestens eines C8-C24-Alkohols.

C8-C24-Alkohole c) dienen in den erfindungsgemäßen Haarbehandlungsmitteln als rückfettende, pflegende Wirkstoffe.

Für die Verwendung in den erfindungsgemäßen Haarbehandlungsmitteln geeignet sind vorzugsweise C8-C24-Monoalkohole c) mit verzweigter oder unverzweigter Kohlenstoffkette. Besonders bevorzugt sind Octylalkohol, Decylalkohol, Laurylalkohol, Myristylalkohol, Cetylalkohol, Isocetylalkohol, Stearylalkohol, Isostearylalkohol, Cetearylalkohol, Arachidylalkohol, Behenylalkohol, Lignocerylalkohol, Palmitoleylalkohol, Oleylalkohol sowie Mischungen davon. Insbesondere bevorzugt sind Laurylalkohol, Myristylalkohol, Cetylalkohol, Isocetylalkohol, Stearylalkohol, Isostearylalkohol, Cetearylalkohol sowie beliebige Mischungen davon.

Der mindestens eine C8-C24-Alkohol c) kann in den erfindungsgemäßen Haarbehandlungsmitteln vorzugsweise in Mengen von 6 bis 33 Gew.-%, mehr bevorzugt 7 bis 32 Gew.-%, besonders bevorzugt von 8 bis 31 Gew.-% und insbesondere von 10 bis 30 Gew.-% eingesetzt werden (bezogen auf das Gesamtgewicht der Haarbehandlungsmittel).

Vorzugsweise enthalten erfindungsgemäße Haarbehandlungsmittel Laurylalkohol, Myristylalkohol, Cetylalkohol, Isocetylalkohol, Stearylalkohol, Isostearylalkohol und/oder Cetearylalkohol in den zuvor genannten Mengen.

Die Erfindung betrifft ferner ein Haarbehandlungsmittel wie zuvor beschrieben, umfassend (bezogen auf das Gesamtgewicht des Haarbehandlungsmittels):
d) 5 bis 35 Gew.-% mindestens C6-C24-Fettsäureesters.

Unter erfindungsgemäß geeigneten C6-C24-Fettsäureestern d) werden beispielsweise verstanden:
- Ester von C₆-C₃₀-Fettsäuren mit C₂-C₃₀-Fettalkoholen verstanden. Bevorzugt sind Monoester von Fettsäuren mit Alkoholen mit 2 bis 24 C-Atomen (Esteröle). Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind 2-Ethylhexylpalmitat (Cegesoft^{®} 24), Isopropylmyristat (Rilanit^{®} IPM), Isononansäure-C16-18-alkylester (Cetiol^{®} SN), Stearinsäure-2-ethylhexylester (Cetiol^{®} 868), Cetyloleat, Glycerintricaprylat, Kokosfettalkohol-caprinat/-caprylat (Cetiol^{®} LC), Cococaprylat (Cetiol^{®} C5), n-Butylstearat, Oleylerucat (Cetiol° J 600), Isopropylpalmitat (Rilanit^{®} IPP), Isopropylisostearat, Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
   Ganz besonders bevorzugt sind Coco Caprylate, Isopropylmyristat, Isopropyl Isostearate oder Mischungen davon.
- mit Ethylenoxid, Propylenoxid oder Mischungen aus Ethylenoxid und Propylenoxid alkoxylierte Esteröle. Die Alkoxylierung kann dabei sowohl am Fettalkoholteil als auch am Fettsäureteil als auch an beiden Teilen der Esteröle zu finden sein. Bevorzugt ist, wenn zunächst der Fettalkohol alkoxyliert wurde und anschließend mit Fettsäure verestert wurde. In der Formel (D4-II) sind allgemein diese Verbindungen dargestellt. 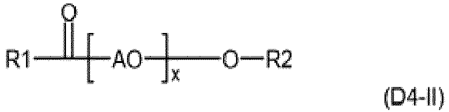 R1 steht hierbei für einen gesättigten oder ungesättigten, verzweigten oder unverzweigten, cyclischen gesättigten cyclischen ungesättigten Acylrest mit 6 bis 30 Kohlenstoffatomen, AO steht für Ethylenoxid, Propylenoxid oder Butylenoxid, X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5, R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Beispiele für eingesetzte Fettsäurenanteile als Rest R1 in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile als Rest R2 in den Esterölen sind Benzylalkohol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen.
   Ein erfindungsgemäß besonders bevorzugtes alkoxyliertes Esteröl ist beispielsweise unter der INCI - Bezeichnung PPG-3 Benzyl Ether Myristate, beispielsweise von der Firma Croda als Handelsprodukt Crodamol^{®} STS erhältlich.
- Dicarbonsäureesterwie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise Glycerincarbonat oder Dicaprylylcarbonat (Cetiol^{®} CC),
- Trifettsäureester von gesättigten und/oder ungesättigten linearen und/oder verzweigten Fettsäuren mit Glycerin, bevorzugt mittelkettige Triglyceride mit einer Kettenlänge von 6 bis 14, vorzugsweise 8 bis 10 Kohlenstoffatomen und besonders bevorzugt mittelkettige Triglyceride aus gesättigten Fettsäuren mit 8 bis 10 Kohlenstoffatomen, wie beispielsweise die unter der INCI-Bezeichnung Caprylic/Capric Triglyceride bekannten Verbindungen, welche beispielsweise von der Firma BASF SE unter der Bezeichnung Myritol^{®} 318 erhältlich sind.
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I) 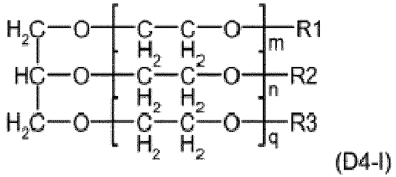 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Es ist erfindungsgemäß möglich und für einige Anwendungsformen bevorzugt, mehr als zwei der zuvor genannten Esteröle gleichzeitig zu verwenden. Bevorzugte Ausführungsformen können beispielsweise Mischungen symmetrischer Kohlensäurediester mit einem oder mehreren alkoxylierten oder nicht alkoxylierten Estern von C₆-C₃₀-Fettsäuren mit C₂-C₃₀-Fettalkoholen und/oder mit Trifettsäureester(n) umfassen. Besonders bevorzugte Ausführungsformen können beispielsweise die Mischungen von
- Dicaprylyl Carbonate und Coco Caprylate
- Dicaprylyl Carbonate und PPG-3 Benzyl Ether Myristate
- Dicaprylyl Carbonate und Isopropylisostearate
- Dicaprylyl Carbonate und Isopropylmyristate
- Dicaprylyl Carbonate und Caprylic/Capric Triglyceride enthalten.

Der mindestens eine C6-C24-Fettsäureester d) kann in den erfindungsgemäßen Haarbehandlungsmitteln vorzugsweise in Mengen von 6 bis 32,5 Gew.-%, mehr bevorzugt 7,5 bis 30 Gew.-%, besonders bevorzugt von 9 bis 27,5 Gew.-% und insbesondere von 10 bis 25 Gew.-% eingesetzt werden (bezogen auf das Gesamtgewicht der Haarbehandlungsmittel). Vorzugsweise enthalten erfindungsgemäße Haarbehandlungsmittel Dicaprylyl Carbonate, Coco Caprylate, PPG-3 Benzyl Ether Myristate, Isopropylisostearate, Isopropylmyristate, Caprylic/Capric Triglyceride oder Mischungen davon in den zuvor genannten Mengen.

Die Erfindung betrifft ferner ein Haarbehandlungsmittel wie zuvor beschrieben, umfassend (bezogen auf das Gesamtgewicht des Haarbehandlungsmittels):
e) 0,1 bis 7 Gew.-% mindestens eines kationischen Tensids.

Kationtenside e) tragen eine positive Ladung in ihrem hydrophilen Teil. Diese positive Ladung bewirkt, dass sich die Tensidmoleküle an der negativ geladenen Haut- und Haaroberfläche anlagern. Auf diese Weise neutralisieren sie die Ladung, verhindern das Fliegen der Haare, wirken glättend, erhöhen den Haarglanz und verbessern die Nasskämmbarkeit. Daneben weisen Kationtenside aufgrund ihrer bakteriziden Wirkung, also einer Bakterien hemmenden Wirkung, in kosmetischen Produkten eine co-konservierende Wirkung auf.

Als kationische Tenside eignen sich in erfindungsgemäßen Haarbehandlungsmitteln prinzipiell alle für die Verwendung am menschlichen Körper geeigneten kationischen oberflächenaktiven Stoffe. Diese sind gekennzeichnet durch mindestens eine wasserlöslich machende, kationische Gruppe, wie z. B. eine quaternäre Ammonium-Gruppe, oder durch mindestens eine wasserlöslich machende, kationisierbare Gruppe, wie z. B. eine Amin-Gruppe und weiterhin mindestens eine lipophile Alkylgruppe mit etwa 6 bis 30 C-Atomen, oder auch durch mindestens eine Imidazol-Gruppe oder mindestens eine Imidazylalkyl-Gruppe.

Im Allgemeinen werden katonische Tenside je nach ihren Strukturmerkmalen in Gruppen aufgeteilt. Besonders geeignet für die Verwendung in den erfindungsgemäßen Zusammensetzungen sind kationische Tenside e) aus mindestens einer der Gruppen der
i. Alkylquats,
ii. Esterquats,
iii. quarternären Imidazolinen,
iv. Amidoaminen und/oder kationisierten Amidoaminen,
v. und/oder Mischungen davon.

Diese spezifisch benannten kationischen Tenside haben in den erfindungsgemäßen Haarbehandlungsmitteln eine als besonders angenehm empfundenen Konditionierwirkung gezeigt. Besonders bevorzugte erfindungsgemäße Haarbehandlungsmittel enthalten als kationische Tenside e) quarternäre Ammoniumverbindungen (Alkylquats), Amidoamine, kationisierte Amidoamine sowie Mischungen hiervon.

Ganz besonders bevorzugte quaternäre Ammoniumverbindungen (Alkylquats) weisen mindestens einem C₈-C₂₄-Alkylrest auf und sind ausgewählt aus quaternären Ammoniumhalogeniden, insbesondere Chloriden, oder aus quaternären Ammoniumalkylsulfaten, wie Methosulfaten oder Ethosulfaten, beispielsweise C₈-C₂₄-Alkyltrimethylammoniumchloride, C₈-C₂₄-Dialkyldimethylammoniumchloride und C₈-C₂₄-Trialkylmethylammoniumchloride wie Cetyltrimethylammoniumchlorid, Stearyltrimethylammoniumchlorid, Behentrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83, Quaternium-87 und Quaternium-91 bekannten Imidazolium-Verbindungen.

Bei Esterquats handelt es sich um kationische Tenside, die sowohl mindestens eine Esterfunktion als auch mindestens eine quartäre Ammoniumgruppe als Strukturelement und weiterhin mindestens einen C8-C24-Alkylrest oder C8-C24-Acylrest enthalten. Bevorzugte Esterquats sind quaternierte Estersalze von Fettsäuren mit Triethanolamin, quaternierte Estersalze von Fettsäuren mit Diethanolalkylaminen und quaternierte Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. N,N-Bis(2-Palmitoyloxyethyl)dimethylammoniumchlorid, Distearoylethyl Dimonium Methosulfate und Distearoylethyl Hydroxyethylmonium Methosulfate sind bevorzugte Beispiele für solche Esterquats. Alkylamidoamine werden üblicherweise durch Amidierung natürlicher oder synthetischer C₈-C₂₄-Fettsäuren und Fettsäureschnitte mit Di-(C₁-C₃)alkylaminoaminen hergestellt. Erfindungsgemäß besonders geeignete Verbindungen aus dieser Substanzgruppe sind beispielsweise unter den INCI-Bezeichnungen Brassicamidopropyldimethylamin, Lauramidopropyldimethylamin, Myristamidopropyldimethylamin, Stearamidopropyldimethylamin, Cocamidopropyldimethylamin, Ricinolamidopropyldimethylamin, Isostearamidopropyldimethylamin, Oleamidopropyldimethylamin, Behenamidopropyldimethylamin und/oder Palmamidopropyldimethylamin bekannte Verbindungen. Insbesondere bevorzugt ist Stearamidopropyl Dimethylamine.

Unter kationisierten Amidoaminen werden vorzugsweise unter den INCI-Bezeichnungen Behenamidopropylethyldimoniumethosulfat, Behenamidopropyl-PG-dimoniumchlorid, Oleamidopropylethyldimoniumethosulfat, Oleamidopropyl-PG-dimoniumchlorid, Cocamidopropylethyldimoniumethosulfat, Cocamidopropyltrimoniumchlorid, Ricinoleamidopropylethyldimoniumethosulfat, Rinoleamidopropyltrimoniumchlorid, Ricinoleamidopropyltrimoniummethosulfat, Stearamidopropylethyldimoniumethosulfat, Stearamidopropyltrimoniummethosulfat, Undecylenamidopropyltrimoniummethosulfat, Lauramidopropyl-PG-dimoniumchlorid und/oder Canolamidopropylethyldimoniumethosulfat, bekannte Verbindungen verstanden.

Das mindestens eine kationische Tensid e) kann in den erfindungsgemäßen Haarbehandlungsmitteln vorzugsweise in Mengen von 0,25 bis 5 Gew.-%, mehr bevorzugt 0,5 bis 5 Gew.-%, besonders bevorzugt von 0,75 bis 5 Gew.-% und insbesondere von 1 bis 5 Gew.-% eingesetzt werden (bezogen auf das Gesamtgewicht der Haarbehandlungsmittel).

In einer bevorzugten Ausführungsform enthalten erfindungsgemäße Haarbehandlungsmittel C8-C24-Alkyltrimethylammonium-, C8-C24-Dialkyldimethylammonium- und/oder C8-C24-Trialkylmethylammonium-Halogenid-, -Ammonium-, -Methosulfat- und/oder Ethosulfatsalze in den zuvor genannten Mengen. Insbesondere bevorzugt enthalten sie mindestens ein unter der INCI-Bezeichnung Cetrimonium Chloride oder Behentrimonium Chloride bekanntes Alkylquat.

In einer weiteren bevorzugten Ausführungsform enthalten erfindungsgemäße Haarbehandlungsmittel mindestens ein unter der INCI-Bezeichnung Palmitamidopropyl Dimethylamine, Stearamidopropyl Dimethylamine, Behenamidopropyl Dimethylamine oder Brassicamidopropyl Dimethylamine bekanntes Aminoamin und/oder eine der kationisierten Formen dieser Amidoamine in den zuvor genannten Mengen.

Für einige Anwendungsformen kann es von Vorteil sein, Mischungen zuvor genannter kationischer Tenside in den zuvor genannten Mengen einzusetzen.

Zusätzlich zu den oben beschriebenen Wirkstoffen a) bis e) können die erfindungsgemäßen Haarbehandlungsmittel weitere Wirkstoffe f) enthalten, welche die Haarkonditionierungswirkung weiter steigern und/oder zur Stabilisierung der Haarbehandlungsmittel betragen.

Bevorzugte Wirkstoffe f) im Rahmen der vorliegenden Erfindung sind
I. von c) und d) verschiedene Rückfettungsmittel und/oder Solubilisierungsmittel,
II. pflanzliche Öle,
III. Pflanzenbuttern,
IV. Wachse und/oder
V. Silikonöle.

Geeignete Komponenten f)I. sind ethoxylierte Glyceridgemische, die sich von Triglyceriden der folgenden Formel ableiten 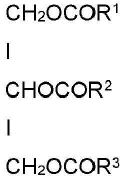 in der R¹CO, R²CO und R³CO unabhängig voneinander für lineare oder verzweigte, gesättigte und/oder ungesättigte Acylreste mit 6 bis 22 und insbesondere 12 bis 18 Kohlenstoffatomen stehen. Typische Beispiele hierfür sind die entsprechenden synthetischen Triglyceride auf Basis der folgenden Fettsäuren: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden jedoch natürliche Fette und Öle eingesetzt, wie beispielsweise Kokosöl, Palmöl, Palmkernöl, Olivenöl, Rapsöl, Rizinusöl und insbesondere Rindertalg. Die natürlichen Triglyceride können dabei naturbelassen sein, vorzugsweise weisen sie jedoch lodzahlen unterhalb von 20 und insbesondere unterhalb von 5 auf, d.h. sie liegen in gehärtetem oder teilgehärtetem Zustand vor.

Typischerweise setzen sich die Partialglyceride wie folgt zusammen:
(a) 50 bis 90, vorzugsweise 60 bis 80 Gew.-% Monoglyceride,
(b) 10 bis 50, vorzugsweise 20 bis 40 Gew.-% Diglyceride und
(c) 0 bis 10, vorzugsweise 3 bis 8 Gew.-% Triglyceride, mit der Maßgabe, dass sich die Mengenangaben zu 100 Gew.-% ergänzen.

In einem zweiten Reaktionsschritt werden die Partialglyceride ethoxyliert. Überwiegend findet die Anlagerung des Ethylenoxids an den primären Hydroxylgruppen der Partialglyceride statt, es können jedoch auch sekundäre Hydroxylgruppen ethoxyliert werden; untergeordnet kommt es sogar zu einer Insertion des Ethylenoxids in die Estercarbonylgruppe. Vorzugsweise werden solche ethoxylierten Glyceridgemische eingesetzt, welche - bezogen den molaren Anteil an Glycerin im Molekül - durchschnittlich 1 bis 80, bevorzugt 3 bis 70 und insbesondere 5 bis 50 Mol Ethylenoxid enthalten. Beispiele für besonders bevorzugte ethoxylierte Glyceridgemische sind die unter den INCI-Bezeichnungen PEG-7 Glyceryl Cocoate und PEG-40 Hydrogenated Castor Oil bekannten Verbindungen.

Ethoxylierte Glyceridgemische können in den erfindungsgemäßen Haarbehandlungsmitteln vorzugsweise in Mengen von 0,1 bis 15 Gew.-%, mehr bevorzugt 0,2 bis 12,5 Gew.-%, besonders bevorzugt von 0,3 bis 10 Gew.-% und insbesondere von 0,4 bis 7,5 Gew.-% eingesetzt werden (bezogen auf das Gesamtgewicht der Haarbehandlungsmittel).

In einer bevorzugten Ausführungsform enthalten die erfindungsgemäßen Haarbehandlungsmittel PEG-7 Glyceryl Cocoate in den zuvor genannten Mengen.

Als weitere geeignete Komponenten f)I. können alkoxylierte, insbesondere ethoxylierte, C6-C24-Fettalkohole dienen, welche verzweigt oder unverzweigt, gesättigt oder ungesättigt sein können. Bevorzugt sind verzweigte C10-C18-Fettalkohole mit einem Ethoxylierungsgrad von 5 bis 40. Insbesondere bevorzugt ist beispielsweise ein unter der INCI-Bezeichnung Isoceteth-20 bekannter ethoxylierter Fettalkohol.

Alkoxylierte C6-C24-Fettalkohole können in den erfindungsgemäßen Haarbehandlungsmitteln vorzugsweise in Mengen von 0,1 bis 15 Gew.-%, mehr bevorzugt 0,2 bis 12,5 Gew.-%, besonders bevorzugt von 0,3 bis 10 Gew.-% und insbesondere von 0,4 bis 7,5 Gew.-% eingesetzt werden (bezogen auf das Gesamtgewicht der Haarbehandlungsmittel).

In einer bevorzugten Ausführungsform enthalten die erfindungsgemäßen Haarbehandlungsmittel Isoceteth-20 in den zuvor genannten Mengen.

Unter pflanzlichen Ölen werden erfindungsgemäß Amaranthsamenöl, Aprikosenkernöl, Arganöl, Avocadoöl, Babassuöl, Baumwollsaatöl, Borretschsamenöl, Camelinaöl, Canolaöl, Cranberryöl, Distelöl, Erdnussöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Kokosnussöl, Kürbiskernöl, Leinöl, Macadamianussöl, Maiskeimöl, Malvenöl, Mandelöl, Mangokernöl, Marulaöl, Melonensamenöl, Mohnöl, Nachtkerzenöl, Olivenöl, Palmöl, Palmkernöl, Pfirsichkernöl, Rambutanöl, Rapssamenöl, Reiskleieöl, Rizinusöl, Sacha Inchi Öl, Safloröl, Sanddornfruchtfleischöl, Sanddornkernöl, Sasanquaöl, Sesamöl, Sheabutter, Sojaöl, Sonnenblumenöl, Teebaumöl, Traubenkernöl, Tsubakiöl, Walnussöl, Weizenkeimöl, Wiesenschaumkrautöl, Wildrosenöl sowie deren Mischungen verstanden.

Pflanzliche Öle können in den erfindungsgemäßen Mitteln vorzugsweise in einer Menge von 0,01 bis 10 Gew.-%, mehr bevorzugt 0,02 bis 7,5 Gew.-%, besonders bevorzugt 0,03 bis 6 Gew.-% und insbesondere 0,05 bis 5 Gew.-% (bezogen auf das Gesamtgewicht des Haarbehandlungsmittels) eingesetzt werden.

Unter erfindungsgemäß geeigneten pflanzlichen Buttern werden vorzugsweise Buttern mit einem Schmelzpunkt im Bereich von 20°C bis 35°C verstanden. Darunter fallen beispielsweise Sheabutter (INCI-Bezeichnung: Butyrospermum-Parkii-(Shea-)Butter), Mangobutter (INCI-Bezeichnung: Mangifera-Indica-(Mango-)Samenbutter), Murumuru-Butter (INCI-Bezeichnung: Astrocaryum-Murumuru-Samenbutter), Kakaobutter (INCI-Bezeichnung: Theobroma-Cacao-(Kakao-)Samenbutter), Cupuacu-Butter (INCI-Bezeichnung: Theobroma-Grandiflorum-Samenbutter), Cocos-Nucifera-(Kokosnuss-)ÖI, Elaeis-Guineensis-(Palm-)Kernöl, Japan-Wachs, synthetisches Japan-Wachs, Ricinus-Communis-(Rizinus-)Samenöl, Simmondsia-Chinensis-(Jojoba-)Butter und Gemische davon. Besonders bevorzugt sind Sheabutter (INCI-Bezeichnung: Butyrospermum-Parkii-(Shea)Butter), Cocos-Nucifera-(Kokosnuss-)ÖI und Mischungen davon.

Pflanzenbuttern können in den erfindungsgemäßen Mitteln vorzugsweise in einer Menge von 0,01 bis 3 Gew.-%, mehr bevorzugt 0,02 bis 2,5 Gew.-%, besonders bevorzugt 0,03 bis 2 Gew.-% und insbesondere bevorzugt 0,05 bis 1,5 Gew.-% (bezogen auf das Gesamtgewicht des Haarbehandlungsmittels) eingesetzt werden.

Unter Wachsen werden erfindungsgemäß bevorzugt aus natürlichen Quellen erhältliche Wachse verstanden, die vorzugsweise einen Schmelzbereich oberhalb von 45°C aufweisen.

Besonders geeignete Wachse im Sinne der vorliegenden Erfindung sind Fruchtwachse, Schalenwachse, Gräserwachse, Blütenwachse und/oder Blattwachse, Bienenwachs oder andere Insektenwachse, Walrat, Wollwachs (Lanolin) und/oder Bürzelfett. Insbesondere bevorzugt sind Beerenwachse, Apfelwachs, Zitronen(schalen)wachs, Reiswachs, Sonnenblumenwachs, Candelillawachs, Jasminwachs, Rosenwachs, Carnaubawachs, Bienenwachs und/oder Wollwachs (Lanolin).

Wachse können den erfindungsgemäßen Haarbehandlungsmitteln vorzugsweise in einer Menge von 0,005 bis 1,00 Gew.-%, bevorzugt von 0,01 bis 0,75 Gew.-%, besonders bevorzugt von 0,025 bis 0,60 Gew.-% und insbesondere in Mengen von 0,05 bis 0,50 Gew.-% (bezogen auf das Gesamtgewicht des Haarbehandlungsmittels) eingesetzt werden.

Unter Silikonölen werden erfindungsgemäß Verbindungen aus der Gruppe der Dialkyl- und Alkylarylsiloxane, besonders bevorzugt aus der Gruppe Polydimethylsiloxane, Cyclopentasiloxane, Cyclohexasiloxane, und Methylphenylpolysiloxane, ganz besonders bevorzugt aus der Gruppe der Polydimethylsiloxane verstanden.

Für die kosmetische Wirkung aber auch für die Konfektionierung und Applikation der erfindungsgemäßen Haarbehandlungsmittel hat es sich als vorteilhaft erwiesen, wenn der Gewichtsanteil der Silikonöle am Gesamtgewicht der Mittel vorzugsweise 0,5 bis 30 Gew.-%, mehr bevorzugt von 1 bis 25 Gew.-%, besonders bevorzugt von 2 bis 20 Gew.-% und insbesondere 3 bis 15 Gew.-% beträgt.

Neben den zuvor beschriebenen Aktivstoffen können die erfindungsgemäßen Haarbehandlungsmittel weitere Inhaltsstoffe enthalten. Die Gruppe dieser weiteren Inhaltsstoffe beinhaltet insbesondere die kosmetisch wirksamen Wirkstoffe, Hilfsstoffe und Zusatzstoffe.

Eine erste Gruppe optionaler Inhaltsstoffe sind die sogenannten Fette oder Öle f), welche bereits an früherer Stelle dieser Anmeldung beschrieben wurden.

Weitere geeignete Wirkstoffe, Hilfsstoffe und Zusatzstoffe sind insbesondere zusätzliche Pflegestoffe. Das Haarbehandlungsmittel kann zum Beispiel mindestens ein Proteinhydrolysat und/oder eines der Derivate davon als Pflegestoff enthalten. Proteinhydrolysate sind Produktgemische, die durch säurekatalysierten, basenkatalysierten oder enzymatisch katalysierten Abbau von Proteinen erhalten werden. Unter dem Begriff "Proteinhydrolysate" werden gemäß der Erfindung ebenso Totalhydrolysate und ebenso einzelne Aminosäuren und Derivate davon sowie Gemische unterschiedlicher Aminosäuren verstanden. Die Molekularmasse der gemäß der Erfindung verwendbaren Proteinhydrolysate liegt zwischen 75, der Molekularmasse für Glycin, und 200.000 Dalton, und die Molekularmasse ist bevorzugt 75 bis 50.000 Dalton und ganz besonders bevorzugt 75 bis 20.000 Dalton.

Als Pflegestoff kann das erfindungsgemäße Haarbehandlungsmittel ebenso mindestens ein Vitamin, ein Provitamin, eine Vitaminvorläufersubstanz und/oder eines der Derivate davon enthalten. Hier werden gemäß der Erfindung Vitamine, Provitamine und Vitaminvorläufersubstanzen bevorzugt, die normalerweise den Gruppen A, B, C, E, F und H zugeordnet sind.

Als Pflegestoff können die erfindungsgemäßen Haarbehandlungsmittel ebenso mindestens einen Pflanzenextrakt, aber ebenso Monosaccharide oder Oligosaccharide und/oder Lipide enthalten. Das hierin offenbarte kosmetische Haarbehandlungsmittel kann ebenso ein Parfüm in einer bevorzugten Menge (bezogen auf das Gesamtgewicht des Haarbehandlungsmittels) von 0,1 bis 3,0 Gew.-%, mehr bevorzugt 0,2 bis 2,5 Gew.-% enthalten.

Durch die Kombination der beschriebenen wesentlichen und fakultativen Inhaltsstoffe ist es möglich, einfach und schnell applizierbare Haarbehandlungsmittel bereitzustellen, welche sich auf die gesamten Haarlängen bis in die Haarspitzen auftragen lassen.

Durch den Kontakt mit Wasser wird das erfindungsgemäße, homogenisierte Haarbehandlungsmittel in eine Creme, eine Lotion oder in ein Gel transformiert, welche(s) weiter in die Haare einmassiert werden kann und den Pflegeeffekt spürbar werden lässt.

Es wurde gefunden, dass die erfindungsgemäßen Haarbehandlungsmittel und deren spezifische Anwendung die Abscheidung von Pflegestoffen auf den Haarfasern begünstigen, so dass ein nachhaltiger Konditionierungseffekt erzielt werden kann.

Damit bei der Lagerung der erfindungsgemäßen Mittel im Ruhezustand keine unerwünschten Eintrübungen an der Phasengrenze und/oder damit erst beim Kontakt mit Wasser die beabsichtigte Transformation in eine Creme, eine Lotion oder ein Gel stattfindet ist es von Vorteil, wenn die erfindungsgemäßen Haarbehandlungsmittel im Wesentlichen frei von zugesetztem Wasser sind. Unter "im Wesentlichen frei" wird verstanden, dass die erfindungsgemäßen Haarbehandlungsmittel (bezogen auf ihr Gesamtgewicht) vorzugsweise einen Maximalgehalt von 15 Gew.-%, mehr bevorzugt 12,5 Gew.-%, besonders bevorzugt 10 Gew.-% und ganz besonders bevorzugt 8 Gew.-% freies Wasser enthalten. Insbesondere bevorzugt wird den erfindungsgemäßen Haarbehandlungsmitteln kein freies Wasser hinzugefügt.

Die erfindungsgemäßen Mittel können dennoch einen kleinen Anteil an Wasser in den zuvor genannten Grenzen enthalten; beispielsweise für den Fall, dass Wasser Bestandteil von Wirkstoffmischungen ist, die den erfindungsgemäßen Haarbehandlungsmitteln hinzugefügt werden können.

Die erfindungsgemäßen Haarbehandlungsmittel umfassen zwei optisch getrennte Schichten, die durch Schütteln homogenisiert werden können, und die sich im Ruhezustand wieder in zwei separate Schichten trennen.

Optimalerweise weisen die erfindungsgemäßen Haarbehandlungsmittel innerhalb von zehn Stunden, vorzugsweise innerhalb von sieben Stunden, besonders bevorzugt innerhalb von fünf Stunden, ganz besonders bevorzugt innerhalb von drei Stunden und insbesondere innerhalb von einer Stunde optisch wieder ihren Ausgangszustand mit zwei Schichten auf.

Für eine schnelle Durch- und Entmischung der beiden Schichten kann es von Vorteil sein, wenn sie geringere Viskositätswerte aufweisen. Unter "geringeren Viskositätswerten" sind im Rahmen der vorliegenden Erfindung vorzugsweise Viskositätswerte der beiden Schichten von jeweils bis zu maximal 1500 mPas, mehr bevorzugt 1000 mPas und insbesondere 500 mPas zu verstehen (gemessen mit Brookfield RVDV II+; 20°C; speed rotation: 20min⁻¹; Spindle No 2; read after 20 sec.). Für eine bessere Erkennbarkeit der Durch- und Entmischung der beiden Schichten kann es weiterhin von Vorteil sein, wenn eine der Schichten transparent ist.

Unter dieser Voraussetzung lässt sich eine ausreichende Durchmischung der beiden Schichten vorzugsweise daran erkennen, dass nur noch eine einzige Phase sichtbar ist, die opak bis milchig trüb sein kann.

Insbesondere bevorzugt ist mindestens eine der beiden Schichten transparent.

Unter "Transparenz" wird im Sinne der vorliegenden Erfindung verstanden, dass die Haarbehandlungsmittel Licht im für Menschen gewöhnlich sichtbaren Bereich nicht streuen. Ebenfalls zur besseren Visualisierung kann eine der beiden Schichten mit einem kosmetisch akzeptablen Farbstoff angefärbt sein.

Es ist weiterhin wünschenswert und bevorzugt, dass die erfindungsgemäßen Haarbehandlungsmittel nach der Homogenisierung der beiden Schichten direkt auf die Haare aufgetragen werden - bevorzugt unter Zuhilfenahme einer Applikationsflasche.

Alternativ können die erfindungsgemäße Zusammensetzung in der Form eines aufgesprühten Haarbehandlungsmittels aufgetragen und auf das Haar aufgesprüht werden.

Das Spray kann ein Pumpspray sein oder das Versprühen kann mit Hilfe von Treibmitteln erreicht werden.

Für die Applikation aus einem Pumpspender ist es von Vorteil, wenn die erfindungsgemäßen Haarbehandlungsmittel frei von Treibmitteln sind.

Geeignete Treibmittel (Treibgase) für erfindungsgemäße Haarbehandlungsmittel, welche als Aerosol versprüht werden, sind Propan, Propen, n-Butan, iso-Butan, iso-Buten, n-Pentan, Penten, isoPentan, iso-Penten, Methan, Ethan, Dimethylether, Stickstoff, Luft, Sauerstoff, Distickstoffoxid, 1,1,1,3-Tetrafluorethan, Heptafluor-n-propan, Perfluorethan, Monochlordifluormethan, 1,1-Difluorethan, genauer gesagt entweder einzeln oder in Kombination. Hydrophile Treibgase, wie etwa Kohlendioxid, können ebenso vorteilhaft verwendet werden, sofern der Anteil an hydrophilen Gasen gering ausgewählt wird und ein lipophiles Treibgas (zum Beispiel Propan/Butan) im Überschuss vorhanden ist. Propan, n-Butan, iso-Butan und Gemischen dieser Treibgase sind besonders bevorzugt. Es hat sich gezeigt, dass die Verwendung von n-Butan als alleiniges Treibgas besonders bevorzugt sein kann. Gefäße aus Metall (Aluminium, Weißblech, Zinn), geschütztem oder nichtsplitterndem Kunststoff oder Glas, das außen mit Kunststoff beschichtet ist, können als Druckgasbehälter verwendet werden; Druckfestigkeit und Bruchfestigkeit, Korrosionsbeständigkeit, leichte Füllbarkeit sowie ästhetische Gesichtspunkte, Handlichkeit, Bedruckbarkeit usw. spielen in ihrer Auswahl eine Rolle.

Vorteilhaft an einem Spray ist die einfache, saubere und zeitsparende Handhabbarkeit, denn die Haarbehandlungsmittel lassen sich aus einem geeigneten Spender durch einfache Betätigung eines Pumpventils als feiner Sprühnebel verteilen und erreichen alle Bereiche der Haare.

Die Hände kommen auf diese Weise nicht mit dem Mittel in Berührung und müssen nach der Anwendung des Mittels nicht gereinigt werden.

Erfindungsgemäße Haarbehandlungsmittel können ressourcenschonend hergestellt, verpackt und transportiert werden, weist keine permanenten Instabilitäten sowie eine Reihe praktischer Vorteile auf. Beispielsweise lassen sie sich einfach und rückstandsarm aus der Verpackung (wie beispielsweise einer Applikationsflasche entnehmen und sehr gut in den Haarfasern verteilen. Vorteilhaft ist weiterhin, dass die Pflegestoffe in den erfindungsgemäßen Haarbehandlungsmitteln nicht in einer festen Matrix eingebunden sind und daher schnell auf die Haarfasern gelangen. Außerdem werden niedrigviskose Produkte als leicht, modern und dennoch pflegend wahrgenommen und die Transformation von "flüssigem Zweiphasenprodukt mit vorzugsweise mindestens einer transparenten Schicht" zu "homogener - üblicherweise trüber - Mischung durch Schütteln" und schließlich zu "weißer Creme oder Lotion" kann besonders gut visualisiert werden.

Die Erfindung kann durch die folgenden nummerierten Aussagen gekennzeichnet sein:
1. Haarbehandlungsmittel für die Pflege von Haaren in Form eines Zweiphasensystems mit zwei separaten Schichten, enthaltend - bezogen auf das sein Gesamtgewicht -
   a) 10-40 Gew.-% mindestens eines C2-C6-Monohydroxyalkohols,
   b) 10 - 60 Gew.-% mindestens eines C2-C12-Polyols,
   c) 5 - 35 Gew.-% mindestens eines C8-C24-Alkohols,
   d) 5 - 35 Gew.-% mindestens eines C6-C24-Fettsäureesters und
   e) 0,1 - 7 Gew.-% mindestens eines kationischen Tensids.
2. Haarbehandlungsmittel nach Aussage 1, enthaltend als C2-C6-Monohydroxyalkohol a) Ethanol, Propanol, Isopropanol oder Mischungen davon.
3. Haarbehandlungsmittel nach Aussage 1, enthaltend Ethanol.
4. Haarbehandlungsmittel nach Aussage 1, enthaltend Propanol.
5. Haarbehandlungsmittel nach Aussage 1, enthaltend Isopropanol.
6. Haarbehandlungsmittel nach einer vorhergehenden Aussage, wobei die Gesamtmenge des oder jedes C2-C6-Monohydroxyalkohols von 11 bis 38 Gew.-% reicht, relativ zu dem Gesamtgewicht des Haarbehandlungsmittels.
7. Haarbehandlungsmittel nach einer vorhergehenden Aussage, wobei die Gesamtmenge des oder jedes C2-C6-Monohydroxyalkohols von 12 bis 37 Gew.-% reicht, relativ zu dem Gesamtgewicht des Haarbehandlungsmittels.
8. Haarbehandlungsmittel nach einer vorhergehenden Aussage, wobei die Gesamtmenge des oder jedes C2-C6-Monohydroxyalkohols von 13 bis 36 Gew.-% reicht, relativ zu dem Gesamtgewicht des Haarbehandlungsmittels.
9. Haarbehandlungsmittel nach einer vorhergehenden Aussage, wobei die Gesamtmenge des oder jedes C2-C6-Monohydroxyalkohols von 15 bis 35 Gew.-% reicht, relativ zu dem Gesamtgewicht des Haarbehandlungsmittels.
10. Haarbehandlungsmittel nach einer vorhergehenden Aussage, enthaltend als C2-C12-Polyol eine Mischung aus
   bi) mindestens einem C2-C6-Diol und
   bii) mindestens einem C2-C12-Polyol, welches mindestens drei Hydroxylgruppen aufweist, in einem Gewichtsverhältnis bi) : bii) von 1 : 10 bis 1 : 3.
11. Haarbehandlungsmittel nach einer vorhergehenden Aussage, enthaltend - bezogen auf sein Gesamtgewicht -
   bi) 1-15 Gew.-% mindestens eines C2-C6-Diols und
   bii) 10-35 Gew.-% mindestens eines C2-C12-Polyols, welches mindestens drei Hydroxylgruppen aufweist.
12. Haarbehandlungsmittel nach einer der Aussagen 10 oder 11, enthaltend als C2-C6-Diol bi)1,2-Propylenglykol, Butan-1,3-diol, Butan-2,3-diol, 1-Methyl-2,4-butandiol, 2-Methyl-2,4-pentandiol, 1,5-Pentandiol, 2-Ethyl-1,3-hexandiol oder beliebige Mischungen davon.
13. Haarbehandlungsmittel nach einer der Aussagen 10 bis 12, enthaltend als C2-C12-Polyol bii) Glycerin, 1,2,4-Pentantriol, 1,3,4-Pentantriol, 1,3,5-Pentantriol, Sorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Diglycerin, Polyglycerin-3, Glucose, Lactose, Maltose, Polyglycerin-4, Dipropylenglykol, Tripropylenglykol oder beliebige Mischungen davon.
14. Haarbehandlungsmittel nach einer vorhergehenden Aussage, enthaltend 1,2-Propylenglykol.
15. Haarbehandlungsmittel nach einer vorhergehenden Aussage, enthaltend Glycerin.
16. Haarbehandlungsmittel nach einer vorhergehenden Aussage, enthaltend 1,2-Propylenglykol und Glycerin.
17. Haarbehandlungsmittel nach einer vorhergehenden Aussage, enthaltend - bezogen auf sein Gesamtgewicht -
   bi) 1 - 15 Gew.-%, vorzugsweise 1,5 bis 14 Gew.-%, besonders bevorzugt 2 bis 12,5 Gew.-% und insbesondere 2,5 bis 10 Gew.-%1,2-Propylenglykol und
   bii) 12,5-34 Gew.-% vorzugsweise 15 bis 32,5 Gew.-%, besonders bevorzugt 17,5 bis 31 Gew.-% und insbesondere 20 bis 30 Gew.-% Glycerin.
18. Haarbehandlungsmittel nach einer vorhergehenden Aussage, enthaltend als C8-C24-Alkohol Octylalkohol, Decylalkohol, Laurylalkohol, Myristylalkohol, Cetylalkohol, Isocetylalkohol, Stearylalkohol, Isostearylalkohol, Cetearylalkohol, Arachidylalkohol, Behenylalkohol, Lignocerylalkohol, Palmitoleylalkohol, Oleylalkohol sowie Mischungen davon.
19. Haarbehandlungsmittel nach einer vorhergehenden Aussage, wobei der C8-C24-Alkohol Laurylalkohol, Myristylalkohol, Cetylalkohol, Isocetylalkohol, Stearylalkohol, Isostearylalkohol und/oder Cetearylalkohol umfasst oder aus diesen besteht.
20. Haarbehandlungsmittel nach einer vorhergehenden Aussage, wobei die Gesamtmenge des oder jedes C8-C24-Alkohols von 6 bis 33 Gew.-% reicht, relativ zu dem Gesamtgewicht des Haarbehandlungsmittels.
21. Haarbehandlungsmittel nach einer vorhergehenden Aussage, wobei die Gesamtmenge des oder jedes C8-C24-Alkohols von 7 bis 32 Gew.-% reicht, relativ zu dem Gesamtgewicht des Haarbehandlungsmittels.
22. Haarbehandlungsmittel nach einer vorhergehenden Aussage, wobei die Gesamtmenge des oder jedes C8-C24-Alkohols von 8 bis 31 Gew.-% reicht, relativ zu dem Gesamtgewicht des Haarbehandlungsmittels.
23. Haarbehandlungsmittel nach einer vorhergehenden Aussage, wobei die Gesamtmenge des oder jedes C8-C24-Alkohols von 10 bis 30 Gew.-% reicht, relativ zu dem Gesamtgewicht des Haarbehandlungsmittels.
24. Haarbehandlungsmittel nach einer vorhergehenden Aussage, wobei der C6-C24-Fettsäureester unter den INCI-Bezeichnungen Dicaprylyl Carbonate, Coco Caprylate, PPG-3 Benzyl Ether Myristate, Isopropyl Isostearate, Caprylic/Capric Triglyceride bekannte Verbindungen umfasst oder aus diesen besteht.
25. Haarbehandlungsmittel nach Aussage 24, enthaltend Dicaprylyl Carbonate und Coco Caprylate.
26. Haarbehandlungsmittel nach Aussage 24, enthaltend Dicaprylyl Carbonate und PPG-3 Benzyl Ether Myristate.
27. Haarbehandlungsmittel nach Aussage 24, enthaltend Dicaprylyl Carbonate und Isopropyl Isostearate.
28. Haarbehandlungsmittel nach Aussage 24, enthaltend Dicaprylyl Carbonate und Caprylic/Capric Triglyceride.
29. Haarbehandlungsmittel nach Aussage 24, enthaltend Dicaprylyl Carbonate und Isopropylmyristate.
30. Haarbehandlungsmittel nach einer vorhergehenden Aussage, wobei die Gesamtmenge des oder jedes C6-C24-Fettsäureesters d) von 6 bis 32,5 Gew.-% reicht, relativ zu dem Gesamtgewicht des Haarbehandlungsmittels.
31. Haarbehandlungsmittel nach einer vorhergehenden Aussage, wobei die Gesamtmenge des oder jedes C6-C24-Fettsäureesters d) von 7,5 bis 30 Gew.-% reicht, relativ zu dem Gesamtgewicht des Haarbehandlungsmittels.
32. Haarbehandlungsmittel nach einer vorhergehenden Aussage, wobei die Gesamtmenge des oder jedes C6-C24-Fettsäureesters d) von 9 bis 27,5 Gew.-% reicht, relativ zu dem Gesamtgewicht des Haarbehandlungsmittels.
33. Haarbehandlungsmittel nach einer vorhergehenden Aussage, wobei die Gesamtmenge des oder jedes C6-C24-Fettsäureesters d) von 10 bis 25 Gew.-% reicht, relativ zu dem Gesamtgewicht des Haarbehandlungsmittels.
34. Haarbehandlungsmittel nach einer vorhergehenden Aussage, enthaltend als kationisches Tensid
   i. Alkylquats,
   ii. Esterquats,
   iii. quarternäres Imidazolin,
   iv. Amidoamine und/oder kationisierte Amidoamine,
   v. und/oder Mischungen davon.
35. Haarbehandlungsmittel nach einer vorhergehenden Aussage, wobei das kationische Tensid Alkylquats umfasst oder aus diesen besteht.
36. Haarbehandlungsmittel nach Aussage 35, wobei das kationische Alkylquat Cetyltrimethylammoniumchlorid umfasst oder aus diesem besteht.
37. Haarbehandlungsmittel nach einer der Aussagen 1 bis 35, wobei das kationische Tensid Behentrimethylammoniumchlorid umfasst oder aus diesem besteht.
38. Haarbehandlungsmittel nach einer der Aussagen 1 bis 34, wobei das kationische Tensid Amidoamine umfasst oder aus diesen besteht.
39. Haarbehandlungsmittel nach Aussage 38, wobei das Amidoamin Stearamidopropyl Dimethylamine umfasst oder aus diesem besteht.
40. Haarbehandlungsmittel nach Aussage 38, wobei das Amidoamin Behenamidopropyl Dimethylamine umfasst oder aus diesem besteht.
41. Haarbehandlungsmittel nach Aussage 38, wobei das Amidoamin Brassicamidopropyl Dimethylamine umfasst oder aus diesem besteht.
42. Haarbehandlungsmittel nach einer vorhergehenden Aussage, wobei die Gesamtmenge des oder jedes kationischen Tensids von 0,25 bis 5 Gew.-% reicht, relativ zu dem Gesamtgewicht des Haarbehandlungsmittels.
43. Haarbehandlungsmittel nach einer vorhergehenden Aussage, wobei die Gesamtmenge des oder jedes kationischen Tensids von 0,5 bis 5 Gew.-% reicht, relativ zu dem Gesamtgewicht des Haarbehandlungsmittels.
44. Haarbehandlungsmittel nach einer vorhergehenden Aussage, wobei die Gesamtmenge des oder jedes kationischen Tensids von 0,75 bis 5 Gew.-% reicht, relativ zu dem Gesamtgewicht des Haarbehandlungsmittels.
45. Haarbehandlungsmittel nach einer vorhergehenden Aussage, wobei die Gesamtmenge des oder jedes kationischen Tensids von 1 bis 5 Gew.-% reicht, relativ zu dem Gesamtgewicht des Haarbehandlungsmittels.
46. Haarbehandlungsmittel nach einer der vorhergehenden, enthaltend ethoxylierte Partialglyceride, ethoxylierte Fettalkohole oder Mischungen davon.
47. Haarbehandlungsmittel nach Aussage 46, enthaltend - bezogen den molaren Anteil an Glycerin im Molekül - durchschnittlich 1 bis 80, bevorzugt 3 bis 70 und insbesondere 5 bis 50 Mol Ethylenoxid.
48. Haarbehandlungsmittel nach einer der Aussagen 46 oder 47, wobei die ethoxylierten Partialglyceride unter den INCI-Bezeichnung PEG-7 Glyceryl Cocoate, PEG-40 Hydrogenated Castor Oil bekannte Verbindungen umfassen oder aus diesen bestehen.
49. Haarbehandlungsmittel nach einer der Aussagen 46 bis 48, enthaltend PEG-7 Glyceryl Cocoate.
50. Haarbehandlungsmittel nach einer der Aussagen 44 bis 49, wobei die Gesamtmenge des oder jedes ethoxylierten Partialglycerids von 1 bis 15 Gew.-% reicht, relativ zu dem Gesamtgewicht des Haarbehandlungsmittels.
51. Haarbehandlungsmittel nach einer der vorhergehenden Aussagen, enthaltend mindestens ein pflanzliches Öl, ausgewählt aus Amaranthsamenöl, Aprikosenkernöl, Arganöl, Avocadoöl, Babassuöl, Baumwollsaatöl, Borretschsamenöl, Camelinaöl, Canolaöl, Cranberryöl, Distelöl, Erdnussöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Kokosnussöl, Kürbiskernöl, Leinöl, Macadamianussöl, Maiskeimöl, Malvenöl, Mandelöl, Mangokernöl, Marulaöl, Melonensamenöl, Mohnöl, Nachtkerzenöl, Olivenöl, Palmöl, Palmkernöl, Pfirsichkernöl, Rambutanöl, Rapssamenöl, Reiskleieöl, Rizinusöl, Sacha Inchi Öl, Safloröl, Sanddornfruchtfleischöl, Sanddornkernöl, Sasanquaöl, Sesamöl, Sheabutter, Sojaöl, Sonnenblumenöl, Teebaumöl, Traubenkernöl, Tsubakiöl, Walnussöl, Weizenkeimöl, Wiesenschaumkrautöl, Wildrosenöl sowie beliebigen Mischungen davon.
52. Haarbehandlungsmittel nach einer der vorhergehenden Aussagen, enthaltend Aprikosenkernöl.
53. Haarbehandlungsmittel nach einer der vorhergehenden Aussagen, enthaltend Marulaöl.
54. Haarbehandlungsmittel nach einer der Aussagen 51 bis 53, wobei die Gesamtmenge des oder jedes Pflanzenöls von 0,01 bis 10 Gew.-% reicht, relativ zu dem Gesamtgewicht des Haarbehandlungsmittels.
55. Haarbehandlungsmittel nach einer vorhergehenden Aussage, enthaltend maximal 15 Gew.-% Wasser (bezogen auf das Gesamtgewicht des Haarbehandlungsmittels).
56. Haarbehandlungsmittel nach einer vorhergehenden Aussage, wobei mindestens eine Schicht im Ruhezustand transparent ist.
57. Haarbehandlungsmittel nach einer vorhergehenden Aussage, wobei beide Schichten im Ruhezustand transparent sind.
58. Haarbehandlungsmittel nach einer vorhergehenden Aussage, wobei mindestens eine Schicht im Ruhezustand gefärbt ist.
59. Haarbehandlungsmittel nach einer der vorhergehenden Aussagen, welches durch Schütteln homogenisiert und in Kombination mit Wasser in eine Creme, eine Lotion oder in ein Gel transformiert wird.
60. Haarbehandlungsmittel nach einer der vorhergehenden Aussagen in der Form eines Rinse-off-Haarkonditioniermittels.
61. Kosmetisches Produkt, umfassend ein Haarbehandlungsmittel nach einer vorhergehenden Aussage.
62. Kosmetisches Produkt nach Aussage 61, wobei das Produkt eine transparente odertranslucente Applikationsflasche umfasst.
63. Kosmetisches Produkt nach Aussage 61, wobei das Produkt einen Pumpspender oder ein Pumpspray umfasst.
64. Kosmetisches Verfahren zur Pflege von Haaren, bei dem ein Haarbehandlungsmittel nach einer der Aussagen 1 bis 60 bis zur Homogenität geschüttelt, anschließend auf die Haare aufgebracht und einmassiert wird, wobei sich bei Wasserkontakt eine Creme, eine Lotion oder ein Gel bildet, welche(s) nach einer Einwirkungszeit von bis zu 10 Minuten mit Wasser ausgespült wird.

### Beispiele

Alle angegebenen Mengen betreffen Gewichtsteile. Die folgenden Formulierungen wurden unter Verwendung bekannter Herstellungsverfahren bereitgestellt.

| | **Beispiel 1** | **Beispiel 2** | **Beispiel 3** |
|---|---|---|---|
| C2-C6-Monohydroxyalkohol | 10-40 | 10-40 | |
| Ethanol, Propanol und/oder Isopropanol | | | 15-35 |
| C2-C12-Polyol | 10-60 | | |
| C2-C6-Diol | | 1-15 | |
| C2-C12-Polyol mit mindestens 3 Hydroxylgruppen | | 10-35 | |
| 1,2-Propylenglycol | | | 1-15 |
| Glycerin | | | 10-35 |
| C8-C24-Alkohol | 5-35 | 5-35 | |
| Laurylalkohol, Myristylalkohol, Cetylalkohol, Isocetylalkohol, Stearylalkohol, Isostearylalkohol und/oder Cetearylalkohol | | | 10-30 |
| C6-C24-Fettsäureester | 5-35 | 5-35 | |
| Dicaprylyl Carbonate, Coco Capryate, PPG-3 Benzyl Ether Myristate, Isopropyl isostearate und/oder Caprylic/Capric Triglyceride | | | 10-25 |
| kationischen Tensid | 0,1 -7 | 0,1-7 | |
| Alkylquat, Amidoamine und/oder kationisiertes Amidoamine | | | 1 - 5 |
| ethoxyliertes Partialglycerid | | 1 - 15 | 1-15 |
| pflanzliches Öl | | 0,01 -10 | 0,01 -10 |
| ggfs. weitere Hilfs- und Zusatzstoffe | ad 100 | ad 100 | ad 100 |

| | **Beispiel 4** | **Beispiel 5** | **Beispiel 6** | **Beispiel 7** | **Beispiel 8** |
|---|---|---|---|---|---|
| **Ethanol** | 15-35 | 15-35 | 15-35 | 15-35 | 15-35 |
| **1,2-Propylenglycol** | 1-15 | 1-15 | 1-15 | 1-15 | 1 - 15 |
| **Glycerin** | 10-35 | 10-35 | 10-35 | 10-35 | 10-35 |
| **Myristylalkohol** | 10-30 | 10-30 | 10-30 | 10-30 | 10-30 |
| **Cetrimonium Chloride** | 1 -5 | 1 -5 | 1 -5 | 1 -5 | 1 -5 |
| **Dicaprylyl Carbonate** | 10-25 | 0-25 | 0-25 | 0-25 | 0-25 |
| **Coco Caprylate** | | 1 -25 | | | |
| **PPG-3 Benzyl Ether Myristate** | | | 1 -25 | | |
| **Isopropyl Isostearate** | | | | 1 -25 | |
| **Caprylic/Capric Triglyceride** | | | | | 1 -25 |
| **PEG-7 Glyceryl Cocoate** | 0-15 | 0-15 | 0-15 | 0-15 | 0-15 |
| **Aprikosenkernöl** | 0-10 | 0-10 | 0-10 | 0-10 | 0-10 |
| **ggfs. weitere Hilfs- und Zusatzstoffe** | ad 100 | ad 100 | ad 100 | ad 100 | ad 100 |

Die beispielhaften Haarbehandlungsmittel verleihen damit behandelten Haaren ein gesundes, gepflegtes Erscheinungsbild und eine angenehme Haptik. Insbesondere lassen sich Haare nach der Behandlung gut entwirren und weisen eine sehr gute Kämmbarkeit im nassen und trockenen Zustand, ein gepflegtes und geschmeidiges Haargefühl bis in die Haarspitzen sowie strahlenden Glanz und Anti-Frizz auf. Eine Beschwerung der Haare konnte auch bei regelmäßiger Anwendung der erfindungsgemäßen Zusammensetzungen nicht beobachtet werden.

Es versteht sich, dass die Erfindung im Rahmen der beigefügten Patentansprüche modifiziert werden kann.

## Patentansprüche

1. Haarbehandlungsmittel für die Pflege von Haaren in Form eines Zweiphasensystems mit zwei separaten Schichten, enthaltend - bezogen auf das Gesamtgewicht des Haarbehandlungsmittels -
a) 10-40 Gew.-% mindestens eines C2-C6-Monohydroxyalkohols,
b) 10 - 60 Gew.-% mindestens eines C2-C12-Polyols,
c) 5 - 35 Gew.-% mindestens eines C8-C24-Alkohols,
d) 5 - 35 Gew.-% mindestens eines C6-C24-Fettsäureesters und
e) 0,1 - 7 Gew.-% mindestens eines kationischen Tensids.

2. Haarbehandlungsmittel nach Anspruch 1, enthaltend als C2-C6-Monohydroxyalkohol a) Ethanol, Propanol, Isopropanol und Mischungen davon.

3. Haarbehandlungsmittel nach einem der vorhergehenden Ansprüche, enthaltend als C2-C6-Monohydroxyalkohol a) Ethanol.

4. Haarbehandlungsmittel nach einem der vorhergehenden Ansprüche, enthaltend als C2-C12-Polyol b) eine Mischung aus
bi) mindestens einem C2-C6-Diol und
bii) mindestens einem C2-C12-Polyol, welches mindestens drei Hydroxylgruppen aufweist,
in einem Gewichtsverhältnis bi) : bii) von 1 : 10 bis 1 : 3.

5. Haarbehandlungsmittel nach einem der vorhergehenden Ansprüche, enthaltend als C2-C12-Polyol b) eine Mischung aus 1,2-Propylenglykol und Glycerin.

6. Haarbehandlungsmittel nach einem der vorhergehenden Ansprüche, enthaltend als C8-C24-Alkohol c) Octylalkohol, Decylalkohol, Laurylalkohol, Myristylalkohol, Cetylalkohol, Isocetylalkohol, Stearylalkohol, Isostearylalkohol, Cetearylalkohol, Arachidylalkohol, Behenylalkohol, Lignocerylalkohol, Palmitoleylalkohol, Oleylalkohol sowie Mischungen davon.

7. Haarbehandlungsmittel nach einem dervorhergehenden Ansprüche, enthaltend als kationisches Tensid
i. Alkylquats,
ii. Esterquats,
iii. quarternärem Imidazolin,
iv. Amidoaminen und/oder kationisierten Amidoaminen,
v. und/oder Mischungen davon.

8. Haarbehandlungsmittel nach einem der vorhergehenden Ansprüche, wobei das kationische Tensid e) Alkylquats umfasst oder aus diesen besteht.

9. Haarbehandlungsmittel nach einem der vorhergehenden Ansprüche, enthaltend als C6-C24-Fettsäureester d) symmetrische Kohlensäurediester, alkoxylierte oder nicht alkoxylierte Ester von C₆-C₂₄-Fettsäuren mit C₂-C₂₄-Fettalkoholen, Glycerin-Trifettsäureester oder beliebige Mischungen davon.

10. Haarbehandlungsmittel nach einem vorhergehenden Ansprüche, wobei der C6-C24-Fettsäureester d) unter den INCI-Bezeichnungen Dicaprylyl Carbonate, Coco Caprylate, PPG-3 Benzyl Ether Myristate, Isopropyl Isostearate, Caprylic/Capric Triglyceride bekannte Verbindungen umfasst oder aus diesen besteht.

11. Haarbehandlungsmittel nach einem der vorhergehenden Ansprüche, enthaltend mindestens ein Anlagerungsprodukt von 1 bis 80 Mol Ethylenoxid an Mono-, Di- und/oder Triester von Glycerin und linearen oder verzweigten, gesättigten oder ungesättigten C8-C24-Carbonsäuren in einem Gewichtsanteil von 0,1 bis 15 Gew.-% am Gesamtgewicht des Haarbehandlungsmittels.

12. Haarbehandlungsmittel nach einem der vorhergehenden Ansprüche, enthaltend zusätzlich mindestens ein pflanzliches Öl.

13. Haarbehandlungsmittel nach einem der vorhergehenden Ansprüche, welches nach der Homogenisierung in Kombination mit Wasser in eine Creme, eine Lotion oder in ein Gel transformiert wird.

14. Haarbehandlungsmittel nach einem der vorhergehenden Ansprüche in der Form eines Rinse-off-Haarkonditioniermittels.

15. Kosmetisches Verfahren zur Pflege von Haaren, **dadurch gekennzeichnet, dass** ein Haarbehandlungsmittel nach einem der Ansprüche 1 bis 14 vor der Anwendung bis zur Homogenität geschüttelt und anschließend auf die Haare aufgebracht und einmassiert wird, wobei sich bei Wasserkontakt eine Creme, eine Lotion oder ein Gel bildet, welche nach einer Einwirkungszeit von bis zu 10 Minuten mit Wasser ausgespült wird.
